(19) Europäisches Patentamt
European Patent Office
Office européen des brevets

(11) Numéro de publication : **0 501 870 A1**

# (12) DEMANDE DE BREVET EUROPEEN

(21) Numéro de dépôt : **92400480.7**

(51) Int. Cl.$^5$ : **A61K 31/265**

(22) Date de dépôt : **25.02.92**

(30) Priorité : **26.02.91 FR 9102254**

(43) Date de publication de la demande : **02.09.92 Bulletin 92/36**

(84) Etats contractants désignés : **AT BE CH DE DK ES FR GB GR IT LI LU NL PT SE**

(71) Demandeur : **SOCIETE CIVILE BIOPROJET**
**30, rue des Francs Bourgeois**
**F-75003 Paris (FR)**

(72) Inventeur : **Schwartz, Jean-Charles**
**9 Villa Seiurat**
**F-75014 Paris (FR)**
Inventeur : **Lecomte, Jeanne-Marie**
**30 rue des Francs-Bourgeois**
**F-75003 Paris (FR)**

(74) Mandataire : **Bernasconi, Jean et al**
**CABINET LEMOINE ET BERNASCONI 13, Boulevard des Batignolles**
**F-75008 Paris (FR)**

(54) **Nouvelles compositions pharmaceutiques, notamment pour le traitement des colopathies fonctionnelles et leur procédé d'obtention.**

(57) Les nouvelles compositions pharmaceutiques, applicables au traitement au long cours du syndrome du côlon irritable, sont caractérisées en ce qu'elles comportent un mélange de dérivés d'amino-acide [R] et de dérivé d'amino-acide [S], notamment énantiomères, de l'acétorphan, le rapport de dérivés [R] par rapport à l'ensemble étant de 65 % ± 15 à 20 %.

EP 0 501 870 A1

Jouve, 18, rue Saint-Denis, 75001 PARIS

La présente invention a trait à de nouvelles compositions ayant une activité pharmaceutique, applicables notamment au traitement des colopathies fonctionnelles ou syndrome du côlon irritable. Elle a également trait à l'utilisation d'une composition déjà connue pour préparer un médicament pour le traitement de ces affections. Elle a également trait à un procédé de traitement de ces affections ainsi qu'à un procédé de préparation de ces nouvelles compositions pharmaceutiques.

Les compositions et médicaments selon l'invention contiennent des dérivés d'amino-acide énantiomères connus comme inihibiteurs de l'enképhalinase, enzyme responsable de la dégradation des enképhalines.

Les deux composés énantiomères de l'acétorphan, esters benzyliques du N [(acétylthiométhyl)-2 oxo-1 phényl-3-propyl] glycine [R] et [S] ont été séparés et leurs propriétés respectives ont été ainsi caractérisées (Demande de brevet EP-A-0.318.377).

Les deux composés [R] et [S] présentent une activité inhibitrice de l'enképhalinase voisine (respectivement, pour l'isomère [R] : 1,7 nM et pour l'isomère [S] : 2,2 nM) mais leur activité inhibitrice de l'A.C.E., enzyme responsable de la conversion de l'angiotensine I en angiotensine II, est très différente, celle-ci résidant essentiellement dans l'isomère [S]. Ce dernier présente d'ailleurs un effet antihypertenseur, démontré chez l'homme au cours d'essais cliniques (Lancet, 1990, 336, pp. 307-308 et demande de brevet EP-A-0.318.377).

Par ailleurs, récemment, l'étude de pharmacocinétique des deux énantiomères à permis de montrer que l'isomère [R], dénué d'effet sur l'A.C.E., avait une durée d'action vis-à-vis de l'enképhalinase environ trois fois moins longue que celle de l'isomère [S] et un effet maximal moins intense (Lecomte et al, J. Eur. Pharmacol., 1990, 179, pp. 65-73).

Le composé [R] a été présenté, dans la demande de brevet EP-A-0.318.377 comme étant utilisable comme médicament des colopathies fonctionnelles ou syndrome du colon irritable.

Le composé [S] a également été indiqué, dans cette demande, comme utile pour des indications périphériques telles que colopathies fonctionnelles mais surtout pour des indications cardiovasculaires dans le traitement de l'hypertension artérielle à des doses faibles. Aucune indication de cette nature ne figure dans le brevet européen EP-A-0.038.758 décrivant l'acétorphan racémique, son procédé de préparation et ses propriétés.

Cependant, l'utilisation du dérivé [S] dans le traitement au long cours du syndrome du colon irritable, maladie chronique, ne pouvait guère être envisagée chez des sujets non hypertendus en raison de ses puissants effets cardiovasculaires et rénaux. Le meilleur candidat au traitement de cette maladie chronique était donc le dérivé [R] mais les déposants ont découvert que celui-ci avait, dans cette application, une durée de vie beaucoup plus courte et une activité maximum plus faible, nécessitant une posologie très augmentée et des prises à intervalles plus rapprochés, compliquant de façon importante le traitement au long cours de cette maladie chronique.

La présente invention se propose de remédier à ces inconvénients.

L'invention a pour objet de nouvelles compositions ayant des applications pharmaceutiques et caractérisées en ce qu'elles comportent un mélange de dérivés d'amino-acide [R] et de dérivés d'amino-acide [S], notamment énantiomères, de l'acétorphan, caractérisées en ce que le rapport de dérivé [R] par rapport à la somme de dérivés [R] + [S] est de 65 % ± 15 à 20 %, à l'exception du mélange à 50 %, connu par le racémique acétorphan.

L'invention a également pour objet l'application de ces compositions pharmaceutiques au traitement du syndrome du côlon irritable ou des colopathies fonctionnelles, ainsi qu'à leur utilisation, y compris pour le mélange racémique acétorphan, pour préparer un médicament contre le syndrome du côlon irritable.

Les médicaments selon l'invention sont de préférence préparés pour une administration par voie orale ou parentérale, en présence d'un véhicule ou excipient usuel. De façon préférée, pour une administration orale, les doses sont conditionnées pour contenir entre 10 et 100 mg de mélange par prise unitaire, à posologie pouvant être, par exemple, de 300 mg par jour en trois prises chez l'adulte.

Conformément à l'invention, le mélange, racémique, est dissous ou dispersé dans l'excipient ou véhicule.

L'invention a également trait à un procédé de traitement du syndrome du côlon irritable se caractérisant par l'administration, de préférence en trois prises unitaires journalières, de doses de 10 à 100 mg, de composition pharmaceutique contenant 65 % ± 20 % et de préférence de 50 à 80 % de dérivés [R] par rapport à la somme [R] + [S].

L'invention a également pour objet un procédé de préparation des nouvelles compositions, caractérisé en ce que l'on réalise le dédoublement de l'acide acétylthiométhyl-2 phényl-3 propanoïque par l'éphédrine (1S-2R), et l'on sépare l'acide résiduel enrichi en isomère [R] que l'on soumet ensuite à une réaction d'estérification.

De préférence, on effectue l'estérification par l'action du glycinate de benzyle sur un chlorure de l'acide obtenu, par exemple, par l'action du chlorure du thionyle.

Les dérivés entrant dans les compositions selon l'invention répondent aux formules suivantes :

Pour les dérivés [R] :

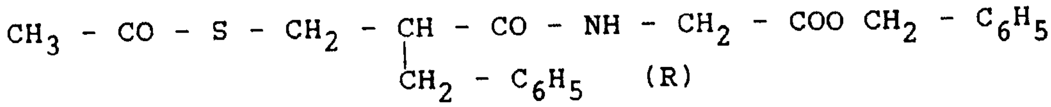

(R)

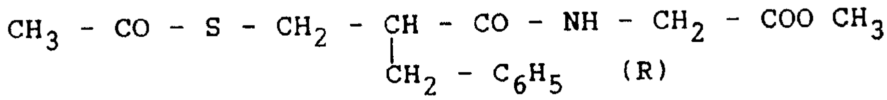

(R)

Pour les dérivés [S] :

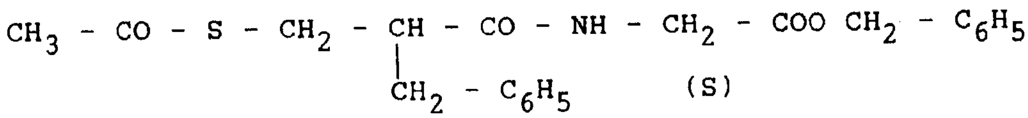

(S)

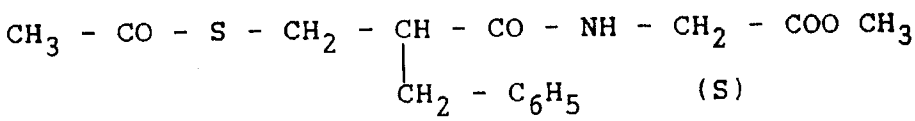

(S)

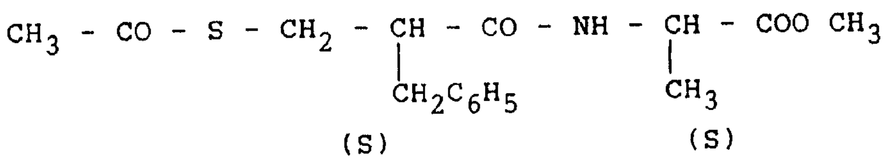

(S) (S)

Les mélanges formant les compositions nouvelles selon l'invention peuvent comprendre, de préférence, les deux énantiomères [R] et [S] correspondants mais ils peuvent également comporter un ou plusieurs énantiomères [R] mélangés à un ou plusieurs énantiomères [S], le pourcentage (en moles) entre les dérivés [R] et [S] étant toujours de 65 plus ou moins 20/35 plus ou moins 20.

Ils peuvent être valablement obtenus selon le procédé de fabrication ci-dessous :

Préparation d'un mélange de [R] acétorphan (65) % et de [S] acétorphan (35%).

I. Schéma réactionnel.

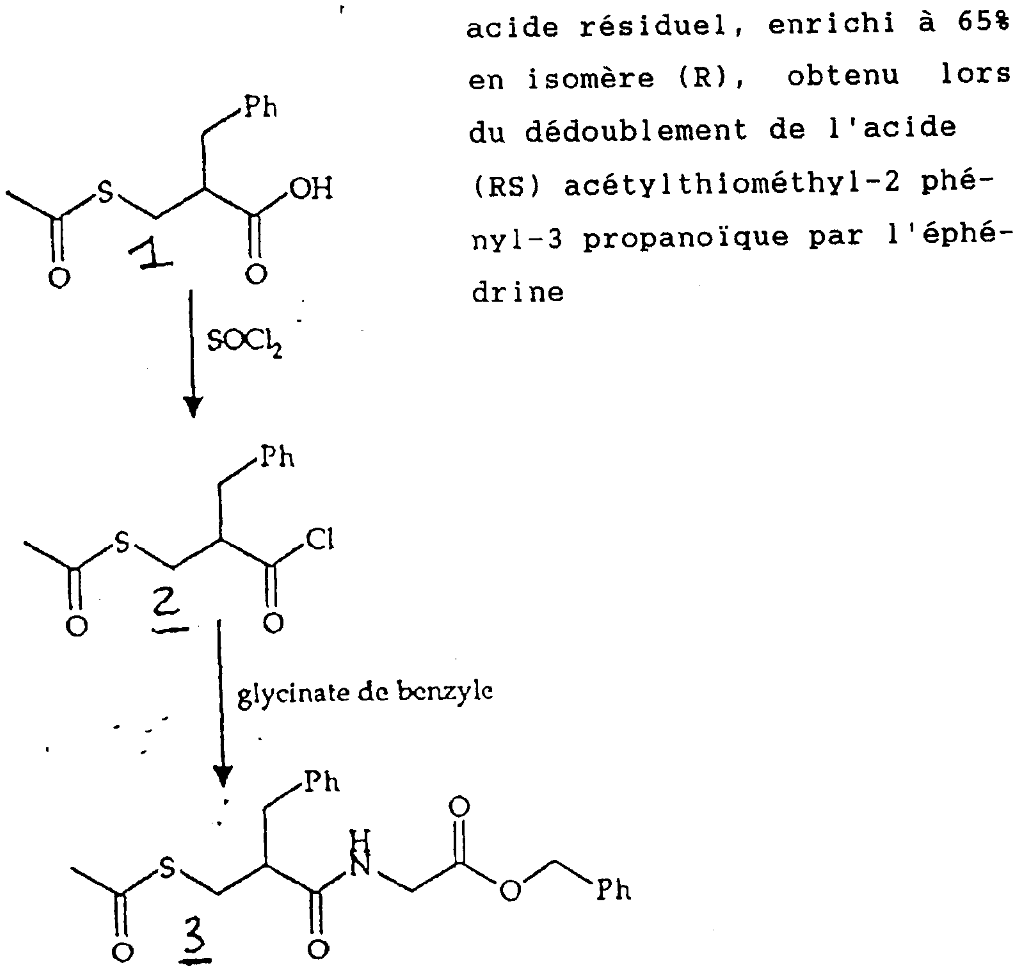


67% d'isomère (R)

II - Synthèse

II.1 - Préparation du chlorure de l'acétylthiométhyl-2 phényl-3 propanoyle 2.

On place dans un ballon, 5,22 g (21,93) mmol) d'acide acétylthiométhyl-2 phényl-3 propanoïque 1 ([α]D$^{25}$ = + 12,5°, c = 1,38 méthanol). On refroidit par un bain glace/eau et ajoute goutte à goutte 3,9 g (32,78 mmol) de chlorure de thionyle. On agite pendant 3 heures à température ambiante.

On évapore l'excès de chlorure de thionyle à l'évaporateur rotatif.
masse obtenue = 5,8 g (rendement quantitatif)
[α]D$^{25}$ = + 10,95° (c = 1,37 dans le chloroforme)
IR : 1790 à 1780, 1695 à 1685 $cm^{-1}$
RMN $^1H$ ($CDCl_3$) : 7,4 à 7,1 (m, 5H) ; 3,5 à 2,8 (m, 5H) ; (s, 3H).

II.2 - Préparation de l'acétorphan 3.

On place dans un tricol muni d'une garde à chlorure de calcium, 3,98 g (11,82 mmol) de glycinate de benzyle sous forme de sel de paratoluène sulfonate et 2,38 g (23,5 mmol) de triéthylamine dans 8 ml de $CH_2Cl_2$. On refroidit par un bain glace/eau. On ajoute goutte à goutte une solution de 3,14 g (11,82 mmol) de chlorure d'acide 2 dans 8 ml de $CH_2Cl_2$. On agite pendant 3 heures à température ambiante.

Le milieu réactionnel est lavé à l'eau (1 fois 10 ml), par une solution aqueuse saturée de $NaHCO_3$ (1 fois 10 ml) et par une solution aqueuse saturée de sodium (1 fois 10 ml). La phase organique est séchée sur sulfate de magnésium, filtrée et concentrée. Le résidu est dissous dans l'isopropanol à chaud (25 ml). La solution est refroidie par un bain glace/eau. Le solide est filtré, lavé refroidie par un bain glace/eau. Le solide est filtré, lavé à l'éther diisopropylique, essoré et séché sous vide sur pentaoxyde de phosphore.

masse obtenue = 3 g (66 %)

$[\alpha]D^{25}$ = + 8,3° (c = 1,34 dans le méthanol)

(Acétorphan optiquement pur : $[\alpha]D^{25}$ = + 23,9° (c = 1,3 méthanol)

Ce produit contient environ 67 % d'isomère (R) et 33 % d'isomère (S).

F = 71°C

RMN $^1H$ ($CHCl_3$): 7,3 (s,5H) ; 7,2(s,5H) ; 5,85(t large, 1H); 5,1 (s,2H) ; 3,95 et 3,9(dd, 2H, J = 5,2 Hz) ; 3,25 à 2,4(m,5H) ; 2,3(s,3H).

A - ETUDE PHARMACOLOGIQUE.

Activité motrice colique.

Chez le chien, en période post-prandiale, le mélange des deux isomères [R] 65 % [S] 35 % potentialise très fortement l'augmentation de la motricité colique, enregistrée par des jauges de contrainte.

Index moteur (évalué par jauge de contrainte)

| Composés | Dose (mg/kg) per os | Avant repas (-2-0 h) | Après repas (0-2 h) | Après repas (2 - 10 h) |
|---|---|---|---|---|
| Témoin | 0 | 6.0 ± 1.2 | 9 ± 1.69 | 10.9 ± 2.3 |
| [R] | 10 | 5.7 ± 0.8 | 16.7* ± 2 | 15.2 ± 2.4 |
| [S] | 5 | 6.3 ± 1 | 19.4* ± 2.8 | 21.7* ± 4.0 |
| [R] 65 % + [S] 35 % | 5 | 6.2 ± 0.9 | 18.9* ± 1.2 | 18.6* ± 4.6 |

* p < 0.01 par rapport à la valeur témoin.

Moyenne + sd de 8 valeurs.

B - ETUDE PHARMACOCINETIQUE

Chez le volontaire sain, l'activité enzymatique plasmatique a été évaluée en fonction du temps pour suivre

l'absorption et la durée de vie des inhibiteurs étudiés, après administration orale d'un dose unique de 30 mg.

Comparaison des inhibitions enképhalinasiques plasmatiques après administration des différents composés à la dose de 30 mg par voie orale.

| | Activité enképhalinase plasmatique (pmol/ml/min) | | | |
|---|---|---|---|---|
| | TO | T30' | T1h | T8h |
| Placebo | 48,4 | 47,4 | 43,4 | 45,7 |
| Isomère [R] | 52,3 | 40,0 | 32,4* | 46,1 |
| Isomère [S] | 53,3 | 28,4* | 21,7* | 37* |
| Mélange [R] 65 % [S] 35 % | 49,1 | 29* | 20,5* | 39* |

* Différence significative par rapport à TO : $p < 0.05$

Il ressort de cette étude que le dérivé [R] a :

1) une durée d'action plus courte que le dérivé [S] et le mélange [R] + [S],

2) un effet maximum [$C_{max}$] plus faible.

C - ETUDE CLINIQUE

Une étude multicentrique chez 420 colopathes fonctionnels a été entreprise avec l'acétorphan [R,S], produit pour lequel les études de toxicité chronique chez l'animal rongeur (rat) et non rongeur (singe) autorisaient des études de longue durée (supérieure à 1 mois). La posologie a été de 300 mg, trois fois par jour.

Le critère principal d'efficacité retenu était la douleur évaluée selon une échelle analogique. Les autres paramètres pris en considération étaient les troubles du transit, qu'il s'agisse de constipation (54,4 % des cas) ou de diarrhée (39 % des cas).

Les résultats sont rapportés ci-dessous:

| % d'amélioration après acétorphan 100 mg X 3 | | | |
|---|---|---|---|
| | 1 mois (420 sujets) | 2 mois (115 sujets) | 3 mois (40 sujets) |
| Douleur | 70 | 84,3 | 87,5 |
| Ballonnement | 60 | 80 | 90 |
| Troubles du transit | 50 | 81,5 | 94 |

Parmi les troubles du transit, ceux-ci étaient indistinctement améliorés.

Ainsi, 20 % des sujets diarrhéiques présentaient une diarrhée objective et importante avant le traitement (d'intensité 2 et 3).

Après un mois de traitement, ils n'étaient plus que 2,4 %. 32 % des sujets constipés souffraient de constipation importante (stades 2 et 3), ils n'étaient plus que 12,7 % après un mois de traitement (dont seulement 0,9 % au stade 3).

On peut donc parler d'un produit "régulateur" des troubles du transit, ce qui jusqu'à maintenant était considéré comme impossible.

L'ensemble des résultats apportés montre que les mélange de [R] acétorphan 65 % ± 15 % et [S] acétorphan 35 % qui s'obtient industriellement à partir des eaux mères résultant de la séparation stéréochimique du [S] acétorphan (Brevet EP-A-0.318.377) présente des particularités très intéressantes par rapport :

– au dérivé [S] qui possède une activité inhibitrice de l'A.C.E. trop importante pour un emploi prolongé en gastroentérologie où des risques d'effets secondaires d'hypotension sont à craindre,

– au dérivé [R] qui possède une durée de vie trop courte pour permettre un emploi à des doses faibles en clinique.

Le mélange [R] acétorphan 65 % ± 15 % et [S] acétorphan 35 % ± 15 % présente le double avantage d'une activité durable même à dose faible, sans effets secondaires de nature cardiovasculaire. Ce mélange présente un intérêt majeur dans le traitement de la colopathie fonctionnelle où, pour la première fois, un médicament est actif sur les troubles du transit quelle qu'en soit la nature (constipation ou diarrhée).

Le composé décrit dans l'invention peut être utilisé comme médicament utile aux doses de 10 à 100 mg par prise unitaire dans le traitement de la colopathie fonctionnelle.

## Revendications

1. Nouvelle composition, ayant des applications pharmaceutiques, caractérisée en ce qu'elle comporte un mélanine de dérivés d'amino-acide [R] et de dérivés d'amino-acide [S], notamment énantiomères de l'acétorphan, et caractérisée en ce que le rapport de dérivés [R] par rapport à la somme de dérivés [R] et [S] est de 65 % ± 15 à 20 %, à l'exception du mélange à 50 %.

2. Composition selon la revendication 1, caractérisée en ce qu'elle comporte au moins un dérivé [R] et au moins un dérivé [S] suivant :
Pour les dérivés [R] :

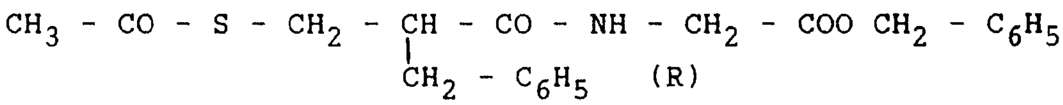

(R)

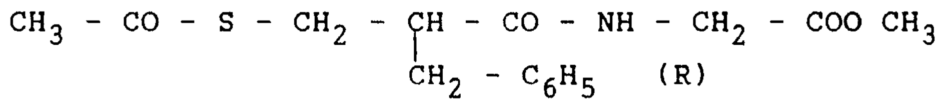

(R)

Pour les dérivés [S] :

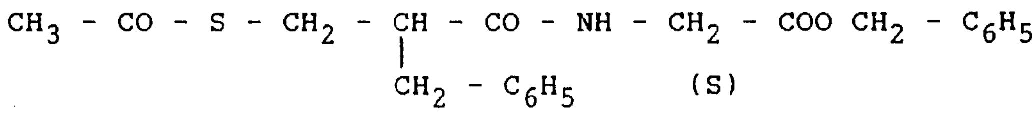

(S)

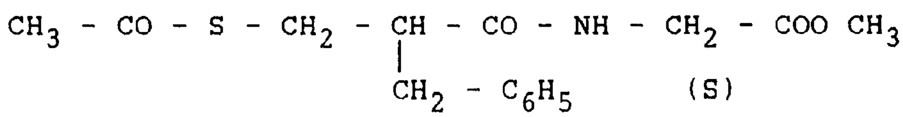

(S)

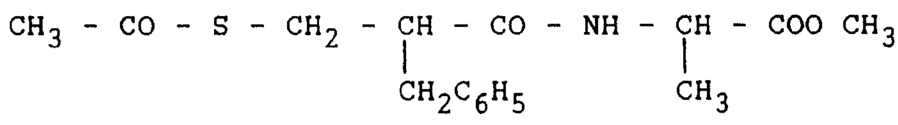

(S) (S)

**3.** Médicament, notamment pour le traitement du syndrome du côlon irritable, caractérisé en ce qu'il comporte une composition selon l'une quelconque des revendications 1 et 2.

**4.** Médicament selon la revendication 3, caractérisé en ce que le rapport de dérivés [R] par rapport à la somme de dérivés est de 65 %.

**5.** Médicament selon l'une quelconque des revendications 3 et 4, caractérisé en ce qu'il est présenté sous doses de 10 à 100 mg de mélange en présence d'un véhicule ou excipient pour administration par voie orale ou parentérale.

**6.** Utilisation d'un mélange équimolaire des énantiomères [R] et [S] de l'acétorphan pour la préparation d'un médicament pour le traitement au long cours du syndrome du côlon irritable.

**7.** Utilisation selon la revendication 6, caractérisée en ce que l'on conditionne ledit mélange en doses de 10 à 100 mg en présence d'un véhicule ou excipient usuel pour l'administration par voie orale ou parentérale.

**8.** Procédé de préparation de nouvelles compositions pharmaceutiques, caractérisé en ce que l'on réalise le dédoublement de l'acide acétylthiométhyl-2 phényl-3 propanoïque par l'éphédrine (1S-2R), et l'on sépare l'acide résiduel enrichi en isomères [R] que l'on soumet ensuite à une réaction d'estérification.

**9.** Procédé selon la revendication 8, caractérisé en ce que l'on réalise l'estérification par action du glycynate de benzyle sur un chlorure de l'acide obtenu, notamment par l'action du chlorure de thionyle.

Office européen des brevets

# RAPPORT DE RECHERCHE EUROPEENNE

Numero de la demande

EP 92 40 0480

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| X,D | EP-A-0 318 377 (SOCIETE CIVILE BIOPROJET) 31 Mai 1989<br>* abrégé *<br>* page 4 - page 5; exemple 1 *<br>----- | 8,9 | A61K31/265 |
| | | | **DOMAINES TECHNIQUES RECHERCHES (Int. Cl.5 )** |
| | | | A61K |

Le présent rapport a été établi pour toutes les revendications

| Lieu de la recherche | Date d'achèvement de la recherche | Examinateur |
|---|---|---|
| LA HAYE | 05 JUIN 1992 | LEHERTE C.F.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)